# EUROPÄISCHE PATENTSCHRIFT

(11) **EP 1 789 803 B1**
(45) Veröffentlichungstag und Bekanntmachung des Hinweises auf die Patenterteilung: **14.07.2010**
(21) Anmeldenummer: 05789386.9
(22) Anmeldetag: 08.09.2005
(51) Int. Cl.: G01N 33/68

(54) **BESTIMMUNG VON FELINEM UND CANINEM PROBNP**
IDENTIFICATION OF FELINE OR CANINE PROBNP
DETERMINATION DU PROBNP CANIN OU FELIN

(30) Priorität: 08.09.2004 AT 15052004
(43) Veröffentlichungstag der Anmeldung: 30.05.2007
(73) Patentinhaber: Biomedica Medizinprodukte GmbH & Co KG, 1210 Wien (AT)
(72) Erfinder: WOLOSZCZUK, Wolfgang, 1040 Wien (AT); HAWA, Gerhard, 1100 Wien (AT)
(74) Vertreter: Sonn & Partner Patentanwälte
(86) Internationale Anmeldenummer: PCT/EP2005/054446
(87) Internationale Veröffentlichungsnummer: WO 2006/027374

(56) Entgegenhaltungen:
- EP-A- 0 648 228
- WO-A-00/45176
- WO-A-00/71576
- US-A- 5 114 923
- US-A1- 2003 069 186
- US-A1- 2004 096 920
- US-B1- 6 586 396
- BIONDO A.W. ET AL.: "Immunohistochemistry of atrial and brain natriuretic peptides in control cats and cats with hypertrophic cardiomyopathy." VETERINARY PATHOLOGY, Bd. 40, Nr. 5, September 2003 (2003-09), Seiten 501-506, XP002355515 ISSN: 0300-9858 in der Anmeldung erwähnt
- JORTANI S.A. ET AL.: "Strategies for developing biomarkers of heart failure." CLINICAL CHEMISTRY, Bd. 50, Nr. 2, Februar 2004 (2004-02), Seiten 265-278, XP002355516 ISSN: 0009-9147 in der Anmeldung erwähnt

## Beschreibung

Die vorliegende Erfindung betrifft ein Verfahren zur Bestimmung von proBNP oder Fragmenten davon in Säugetieren.

Herzerkrankungen spielen nicht nur beim Menschen eine bedeutende Rolle, auch Tiere, im Speziellen Haustiere, wie Hunde und Katzen, sind von diesen Erkrankungen betroffen. Studien habe ergeben, dass beispielsweise jedes zehnte Hundeherz funktionsgestört ist. Die dabei auftretenden Herzerkrankungen betreffen beispielsweise die Herzklappen und den Herzmuskel. Da das Herz in der Lage ist Funktionsstörungen zunächst durch Mehrarbeit auszugleichen, bleibt eine solche Erkrankung zumeist verborgen, was zur Folge hat, dass sich durch die vermehrte Herzbelastung der Zustand des Herzens verschlechtert. Die aus Herzkrankheiten resultierenden Symptome, wie Müdigkeit, Kreislaufschwäche, Abgeschlagenheit, sind meist dann erkennbar, wenn das Herz des Haustieres die Schwäche nicht mehr kompensieren kann. In einem solchen Fall ist die Herzerkrankung bereits so weit fortgeschritten, dass eine vollständige Heilung kaum mehr möglich ist.

Chronische Herzklappen- und Herzmuskelveränderungen sind in der Regel nicht heilbar, wobei aber durch medikamentösen Einsatz das weitere Fortschreiten der Herzerkrankung verlangsamt werden kann. Aus diesem Grund ist es wichtig, eine Frühdiagnose für auftretende Herzerkrankungen zu stellen. Routinemäßig werden hierfür vor allem physikalische Methoden, wie das Abhören der Herztöne, die Aufnahme eines Elektrokardiogramms, Röntgen- und Ultraschalluntersuchungen, eingesetzt. Diese Untersuchungsmethoden weisen vor allem den Nachteil auf, dass sie erst dann durchgeführt werden können, wenn bereits sichtbare bzw. hörbare Schäden am Herzen direkt erkennbar werden. Des Weiteren benötigen physikalische Untersuchungsmethoden geeignete und in der Regel teure Vorrichtungen, um eine entsprechende Diagnose durchzuführen.

Die am häufigsten vorkommenden Herzkrankheiten beispielsweise bei Hunden sind Herzdekompensation und dilatative Kardiomyopathie, welche vorwiegend große Tiere betrifft. Dilatative Kardiomyopathie ist eine Herzerkrankung, die zu einer Vergrößerung der Herzkammern bei normal dicker Wand führt, wobei diese Vergrößerung rasch eine Herzschwäche im betroffenen Tier zur Folge hat. Durch die Zugabe von Taurin in Futtermitteln konnte das Risiko an dilatativer Kardiomyopathie zu erkranken, signifikant reduziert werden. Bei einer der dilatativen Kardiomyopathie verwandten Krankheit, der restriktiven Kardiomyopathie, welche häufig in älteren Katzen beobachtet wird, ist eine kontinuierliche Abnahme der Herzfunktion mit einer reduzierten Pumpfähigkeit beobachtbar. Die bei Katzen am häufigsten vorkommende Herzerkrankung ist hypertrophe Kardiomyopathie. Diese Herzmuskelerkrankung führt zu einer Verdickung der Herzwand und einer daraus resultierenden reduzierten Fähigkeit, die Herzkammern mit Blut zu füllen. Das führt zu einer Anhäufung von Blut in der linken Herzkammer und zu einer stark reduzierten Menge an Blut, die durch den Körper gepumpt wird.

Bei vielen Herzerkrankungen, wie z.B. Herzinsuffizienz, dilatativer Kardiomyopathie, hypertropher Kardiomyopathie, linksventrikulärer Hypertrophie und Dysfunktion, wird ein Peptidhormon, das sogenannte BNP (brain natriuretic peptide) ausgeschüttet. Dieses Hormon bewirkt die Ausscheidung von Flüssigkeit über die Niere und reguliert somit das Herz-Kreislauf-System. Da dieses Peptid im Herzen produziert wird und bei Überlastung und Überfüllung des Herzens vermehrt produziert wird, ist die Bestimmung des BNP-Spiegels im Blut ein geeignetes Mittel zur Beurteilung von Herzschwäche.

BNP wie auch andere natriuretische Peptide spielen bei der Regulierung des Wasserhaushaltes und des Blutdrucks eine bedeutende Rolle. Wenn die Herzwand gedehnt wird, schüttet diese vermehrt BNP aus, was zu einer Ausscheidung von Natrium und Flüssigkeit über die Niere und zu einer Erweiterung der Blutgefäße führt, welche in Summe den Blutdruck und die Füllung des Herzens zu senken vermögen. BNP wird von den Herzmuskelzellen als proBNP synthetisiert, welches schließlich in n-terminales proBNP und BNP gespalten wird. Beide Teile des BNPs werden an das Blut abgegeben und können darin bestimmt werden.

Mit Herzerkrankungen bei Tieren beschäftigen sich unter anderem folgende einschlägigen Veröffentlichungen: Bright JM and Cali JV, J Am Vet Med Assoc 2000, 216:1110-4; Guglielmini C, Vet Res Commun 2003, 27 Suppl 1:555; Boswood A et al., J Small Anim Pract 2003, 44:104-8; Takemura N et al., J Vet Med Sci 2003, 65:1265-7; MacDonald KA et al., J Vet Intern Med 2003, 17:172-7; Greco DS et al., Can Vet J 2003, 44:293-7; Monnet E et al., J Am Vet Med Assoc 1997, 211:569-72; Hamlin RL et al., J Vet Intern Med 1996, 10:85-7; Gaschen L et al., J Vet Intern Med 1999, 13:346-56.

Im Stand der Technik ist bereits eine Vielzahl an Verfahren bekannt, mit deren Hilfe humanes proBNP bzw. deren Fragmente im Serum eines Individuums detektiert werden kann. Beispielhaft seien hierin die EP 0 648 228 B1, WO 03/87819 und FR 2 843 396 erwähnt.

In der US 2004/0018577 ist ein Immunoassay der mindestens drei Antikörper umfasst, welche alle an unterschiedlichen Epitopen eines Analyten zu binden vermögen offenbart. Die dabei zu detektierenden Analyten betreffen insbesondere die Detektion von Markern betreffend Herzerkrankungen, wobei unter anderem auch BNP und proBNP detektiert werden können.

Biondo A.W. et al. (Vet.Pathol..2003, 40(5):501-506) beschreiben ein Verfahren zum Nachweis von ANP und BNP in Katzen mittels polyklonaler Antikörper, die gegen ein Peptid des ANP, welches die Aminosäuren 1 bis 28 umfasst, bzw. gegen ein Peptid, welches die Aminosäuren 43 bis 56 von proBNP umfasst, gerichtet sind.

In der EP 1 016 867 A1 wird ein Immunoassay für den Nachweis von preproBNP in Säugetieren beschrieben. Dabei werden Antikörper verwendet, die gegen Peptide, umfassend die Aminosäuren 27 bis 102, 73 bis 102 und 27 bis 64 von humanem BNP, gerichtet sind.

Jortani S.A. et al. (Clin.Chem.2003, 50(2):265-278) beschreiben die Verwendung von BNP und dessen prepro- und pro-Formen als mögliche Marker für Herzerkrankungen. In diesem Artikel werden keine bevorzugten Peptidregionen von BNP erwähnt, die sich eignen würden, Herzerkrankungen bei Hunden und Katzen nachzuweisen. Vergleichbar dazu wird auch in der WO00/71576 die Verwendung von BNP als Marker beschrieben.

In der WO 2000/35951 werden mehrere Peptide geoffenbart, gegen welche Antikörper hergestellt werden können, die sich in einem Verfahren zur Diagnose von Herzerkrankungen eignen. Dabei werden drei Peptide, umfassend die Aminosäuren 1 bis 13, 37 bis 49 und 65 bis 76 des humanen Nt-pro-BNP-Proteins geoffenbart, welche auch zur Herstellung von Antikörpern, die gegen diese Peptide gerichtet sind, herangezogen werden können.

Die US 6,586,396 betrifft ein Verfahren zur Behandlung von Menschen und Hunden mit Peptiden, welche eine natriuretische Aktivität aufweisen. Dabei wird humanes bzw. canines BNP subkutan verabreicht. Ferner können die in der US-Schrift geoffenbarten BNPs bzw. Fragmente davon zur Herstellung von Antiseren oder monoklonalen Antikörpern, die wiederum in Immunoassays eingesetzt werden könnten, verwendet werden.

Vergleichbar zur US 6,586,396 werden auch in der US 5,114,923 Teile der Aminosäuresequenz des caninen BNPs geoffenbart.

In der US 2004/096920 ist ein ELISA geoffenbart, in dem verschiedene polyklonale Antikörper, die gegen unterschiedliche Bereiche des humanen BNPs gerichtet sind, verwendet werden können.

Die WO 00/45176 betrifft ein Verfahren zum Nachweis von N-terminalen proBNP in einer Probe mit mindestens zwei Antikörpern, die in der Lage sind verschiedene Epitope des N-terminalen proBNPs zu erkennen.

In der US 2003/069186 werden chimäre Proteine geoffenbart, die unter anderem N-terminale NP-Fragmente umfassen können.

Des Weiteren gibt es am Markt mehrere Testkits zur Detektion von humanem proBNP bzw. deren Fragmente (z.B. von Roche und Biomedica). Nichtsdestotrotz gibt es kein bekanntes Verfahren, mit dessen Hilfe spezifisch proBNP in tierischen Proben bestimmt werden kann. Daher und aufgrund der kostenintensiven und aufwendigen physikalischen Untersuchungen von Tieren ist es Aufgabe der vorliegenden Erfindung, geeignete Mittel zur Bestimmung von proBNP bzw. dessen Fragmenten zur Verfügung zu stellen.

Daher sieht die vorliegende Erfindung ein Verfahren zur Bestimmung von felinem oder caninem proBNP umfassend die Schritte:
- Bereitstellen einer felinen oder caninen Probe,
- Inkontaktbringen der Probe mit mindestens einem Antikörper, der bei der Bestimmung von felinem proBNP an mindestens ein Epitop im Bereich der Aminosäuren 20-42 oder im Bereich der Aminosäuren 57-80 des felinen proBNP und bei der Bestimmung von caninem proBNP oder Fragmenten davon an mindestens ein Epitop im Bereich der Aminosäuren 20 bis 86 des caninen proBNP's bindet, und
- Bestimmen der Anwesenheit und/oder Konzentration des in der Probe vorhandenen felinen oder caninen proBNP's, vor.

Es wurde festgestellt, dass ein Antikörper, der an ein Epitop in den offenbarten Bereichen des felinen bzw. caninen proBNP binden kann, sich sehr gut eignet, um proBNP spezifisch zu bestimmen.

Es wird darauf hingewiesen, dass die hierin offenbarten felinen und caninen proBNP-Sequenzen beispielhaft für die Familie der Felidae bzw. der Canidae herangezogen wurden, womit einzelne von diesen hierin offenbarten Sequenzen abweichende Aminosäuren in den proBNP Sequenzen von Tieren anderer Gattungen dieser Familien ebenfalls unter die hierin offenbarten Sequenzen fallen, sofern diese abweichenden Aminosäuren nicht die Epitope der hierin offenbarten Antikörper in der Weise betreffen, dass eine spezifische Bindung nicht mehr ermöglicht wird. Die Aminosäuresequenzen, die hierin offenbart sind, sind in öffentlichen Datenbanken (z.B. Swiss-Prot: canines BNP - P16859 und felines BNP - Q9GLK4) publiziert.

Die im erfindungsgemäßen Verfahren verwendeten Proben umfassen flüssige Proben, wie beispielsweise Blut, Urin, aber auch Gewebeproben, wie beispielsweise Gewebeschnitte der Herzmuskulatur oder des Gehirns. Je nach Bedarf können die Proben entsprechend aufbereitet werden, um beispielsweise das spätere in Kontakt Bringen der Probe mit den erfindungsgemäßen Antikörpern zu erleichtern bzw. zu ermöglichen. So können aus Blutproben Fraktionen enthaltend proBNP bzw. Fragmente davon, bereitgestellt werden oder aber auch Gewebeproben können beispielsweise homogenisiert und ebenfalls von nicht-proteinhaltigen Fraktionen abgetrennt werden.

Die Bindung von mindestens einem Antikörper an ein Epitop des felinen oder caninen proBNP in der Probe bedeutet, dass der Antikörper in der Lage ist, ein Epitop in einem definierten Sequenzbereich eines spezifischen Proteins zu binden, wobei der mindestens eine Antikörper nicht in der Lage ist Epitope des Proteins außerhalb des definierten Bereiches spezifisch zu binden.

Erfindungsgemäß kann ein Antikörper der ein Epitop zu binden vermag eingesetzt werden um proBNP oder Fragmente davon zu bestimmen. Dennoch kann es von Vorteil sein mehrere (z.B. zwei, drei, vier oder fünf) Antikörper, welche verschiedene Epitope des proBNP zu binden vermögen, einzusetzen.

Die Anwesenheit bzw. die Konzentration des in der Probe befindlichen felinen oder caninen proBNP oder Fragements davon kann durch im Stand der Technik bekannte Methoden festgestellt werden. Beispielhaft sei hierbei die Durchführung von Enzym-Immunoassays (z.B. ELISA) bei flüssigen Proben bzw. immunhistochemischen Verfahren bei Gewebeproben erwähnt.

"Antikörper" gemäß der vorliegenden Erfindung umfassen auch Fragmente von Antikörpern, die in der Lage sind ein erfindungsgemäßes Epitop zu erkennen. So kann ein Antikörper beispielsweise lediglich aus dem F(ab)-Teil bestehen, der die antigenbindende Seite aufweist. Diese Antikörperfragmente können des Weiteren Teil eines bispezifischen Antikörpers oder eines "Heterominibodies" (siehe z.B. EP 1 100 830 B1) sein.

"proBNP oder deren Fragmente" umfassen erfindungsgemäß alle proBNP Bruchstücke, die in vivo entstehen (z.B. Nt-proBNP) oder in vitro erzeugt werden (z.B. durch Versetzen einer Probe mit Protease oder chemischen Substanzen wie CNBr), und die erfindungsgemäßen Epitope aufweisen.

Gemäß einer bevorzugten Ausführungsform bindet der mindestens eine Antikörper an mindestens ein Epitop im Bereichder Aminosäuren 25-35 oder im Bereich der Aminosäuren 60-80 des felinen proBNP.

Es wurde gezeigt, dass vor allem die oben angeführten Aminosäurenbereiche des felinen proBNP Epitope aufweisen, die eine spezifische Bindung von Antikörpern erlauben.

In einem Verfahren gemäß der vorliegenden Erfindung können mehrere Antikörper, die mehrere unterschiedliche Epitope an felinen bzw. caninen proBNP spezifisch zu binden vermögen, eingesetzt werden. Aus diesem Grund kann mindestens ein Antikörper, der an mindestens ein Epitop zu binden vermag, erfindungsgemäß eingesetzt werden. Weiters sei hierbei angemerkt, dass die hier angegebenen Aminosäurebereiche nicht nur ein Epitop, sondern je nach Größe mehrere Epitope umfassen können. Somit umfasst das erfindungsgemäße Verfahren die Verwendung einer Kombination mehrerer Antikörper, die an mindestens ein Epitop spezifisch binden können.

Erfindungsgemäß bindet bei der Bestimmung von caninen proBNP der mindestens eine Antikörper an mindestens ein Epitop im Bereich der Aminosäuren 25-41 oder im Bereich der Aminosäuren 55-65 oder im Bereich der Aminosäuren 74-86 des caninen proBNP.

Antikörper, die Epitope in diesen Bereichen erkennen, eignen sich besonders gut zur Bestimmung von proBNP bzw. dessen Fragmente in einer Probe caninem Ursprungs.

Gemäß einer bevorzugten Ausführungsform umfasst das mindestens eine Epitop mindestens drei, mindestens vier, mindestens fünf, mindestens sechs, mindestens sieben, mindestens acht, mindestens neun, mindestens zehn Aminosäuren.

Gemäß einer bevorzugten Ausführungsform ist der mindestens eine Antikörper polyklonal und/oder monoklonal.

Die in einem erfindungsgemäßen Verfahren eingesetzten Antikörper können sowohl polyklonal als auch monoklonal sein. Zur Herstellung dieser Antikörper werden Peptidfragmente umfassend die hierin offenbarten Aminosäurebereiche des felinen bzw. des caninen proBNP verwendet. Diese Peptidfragmente können entweder synthetisch (Merrifield R.P., 1963, J Am Chem Soc 85, 2000, 149), rekombinant oder durch chemischen oder enzymatischen Abbau von proBNP rekombinanten oder nativen Ursprungs hergestellt werden. Die daraus gewonnenen Peptide werden abhängig von ihrer Größe an einen immunogenen Träger (z.B. KLH) gebunden oder direkt zur Herstellung von polyklonalen oder von monoklonalen Antikörpern (z.B. Köhler G. and Milstein C., 1975, Nature 256:495; Galfre et al., 1977, Nature 266:550) eingesetzt. Erfindungsgemäß können die Antikörper auch rekombinant hergestellt werden. Verfahren zur Herstellung von rekombinanten Antikörpern sind dem Fachmann hinreichend bekannt (siehe z.B. Sambrook et al., Molecular Cloning, A Laboratory Manual, Cold Spring Harbor, Laboratory Press, 2001).

Gemäß einer weiteren bevorzugten Ausführungsform bindet an den mindestens einen Antikörper oder an das mindestens eine Epitop mindestens ein weiterer Antikörper, wodurch beispielsweise die Ausführung des erfindungsgemäßen Tests als Sandwich-Assay ermöglicht wird.

Die Bindung eines weiteren Antikörpers an den mindestens einen Antikörper ermöglicht es, diesen und indirekt das an den mindestens einen Antikörper gebundene Epitop qualitativ bzw. quantitativ zu bestimmen. Bindet der mindestens eine weitere Antikörper an das mindestens eine Epitop, ist es möglich, die Bindung des mindestens einen Antikörpers an das mindestens eine Epitop, wenn beispielsweise der mindestens eine Antikörper an eine Festphase immobilisiert ist, qualitativ bzw. quantitativ über ein Enzymimmunoassay zu bestimmen.

Vorzugsweise ist der mindestens eine Antikörper und/oder der mindestens eine weitere Antikörper markiert.

Der mindestens eine Antikörper und/oder der mindestens eine weitere Antikörper ist dabei mit Enzym, wie Peroxidase, insbesondere Meerrettich-Peroxidase, Biotin, Fluoreszenzfarbstoff, insbesondere Fluoreszein (FITC, DFTF), R-Phycoerythrin (PE), Peridiniumchlorophyll-Protein (PerCP) und Tandemkonjugate wie PE-Cy5 oder PE-Texas-Rot, Goldkolloid oder Radionuklide markiert.

Durch die Markierung eines der beiden Antikörper ist es möglich, durch eine Sekundärreaktion oder aber auch direkt die Anwesenheit bzw. Konzentration des markierten Antikörpers gebunden an das mindestens eine Epitop zu bestimmen. Die Antikörper selbst könnten wiederum durch Protein A Konjugate (z.B. Protein A Gold Konjugat) detektiert werden.

Gemäß einer bevorzugten Ausführungsform ist der mindestens eine Antikörper oder der mindestens eine weitere Antikörper an eine Festphase gebunden.

Durch die Bindung des mindestens einen Antikörpers oder des mindestens einen weiteren Antikörpers können beispielsweise Antikörperchips, beschichtete Mikrotiterplatten oder Lateral Flow Devices hergestellt werden, die in einer Vielzahl von Verfahren eingesetzt werden können.

Vorzugsweise erfolgt die Bestimmung von felinem oder caninem proBNP oder Fragmenten davon durch ein Verfahren ausgewählt aus der Gruppe bestehend aus Radioimmunassay, Immunbindungsassay, Westernblot, Immunhistochemie, Enzymimmunoassay, Lateral Flow Device (LFD, Teststreifen) und Kombinationen davon.

Die oben erwähnten Verfahren sind dem Fachmann hinreichend bekannt. Ein Überblick über diese Verfahren kann beispielsweise in "Bioanalytik" (Lottspeich und Zorbas, Spektrum Verlag 1998) gefunden werden. Lateral Flow Devices (LFD, Teststreifen) sind beispielsweise in der WO 02/059567 offenbart.

Gemäß einem weiteren Aspekt betrifft die vorliegende Erfindung Antikörper oder Antikörpermischungen, die an mindestens ein Epitop im Bereich der Aminosäuren 20-42 und/oder im Bereich der Aminosäuren 57-80 des felinen proBNP binden.

Gemäß einer weiteren bevorzugten Ausführungsform binden die Antikörper oder Antikörpermischungen an mindestens ein Epitop im Bereich der Aminosäuren 25-35 oder im Bereich der Aminosäuren 60-80 des felinen proBNP.

Gemäß einem weiteren Aspekt betrifft die vorliegende Erfindung einen Antikörper oder eine Antikörpermischung, die an mindestens ein Epitop im Bereich umfassend die Aminosäuren 20-86 des caninen proBNP binden.

Vorzugsweise bindet der Antikörper oder die Antikörpermischung an mindestens ein Epitop im Bereich der Aminosäuren 25-41 oder im Bereich der Aminosäuren 55-65 oder im Bereich der Aminosäuren 74-86 des caninen proBNP.

Gemäß einer bevorzugten Ausführungsform bindet der Antikörper oder die Antikörpermischung an ein Epitop, das mindestens drei, mindestens vier, mindestens fünf, mindestens sechs, mindestens sieben, mindestens acht, mindestens neun, mindestens zehn Aminosäuren umfasst. Die erfindungsgemäßen Epitope sind vorzugsweise höchstens 40, höchstens 35, höchstens 30, insbesondere höchstens 25, höchstens 20 oder höchstens 15 Aminosäuren lang.

Ein weiterer Aspekt der vorliegenden Erfindung betrifft ein Peptid, welches die Aminosäuren 20-42 und/oder im Bereich der Aminosäuren 57-80 des felinen proBNP aufweist.

Gemäß einer weiteren Ausführungsform weist das Peptid die Aminosäuren 25-35 und/oder im Bereich der Aminosäuren 45-55 und/oder im Bereich der Aminosäuren 60-80 des felinen proBNP auf.

Ein weiterer Aspekt der vorliegenden Erfindung betrifft ein Peptid, das die Aminosäuren 20-86 des caninen proBNP aufweist.

Vorzugsweise weist das Peptid die Aminosäuren 25-41 und/oder die Aminosäuren 55-65 und/oder die Aminosäuren 74-86 des caninen proBNP auf.

Gemäß einer bevorzugten Ausführungsform wird das Peptid chemisch synthetisiert oder von einer Probe isoliert bzw. rekombinant hergestellt.

Um das Epitop aus einem Peptid, welches aus einer Probe isoliert bzw. rekombinant hergestellt wurde, entsprechend herzustellen, kann dieses mit an sich bekannten enzymatischen bzw. chemischen Methoden weiterverarbeitet werden.

Ein weiterer Aspekt der vorliegenden Erfindung betrifft die Verwendung eines erfindungsgemäßen Antikörpers oder einer Antikörpermischung zur Bestimmung von felinem oder caninem proBNP in dem erfindungsgemäßen Verfahren.

Die erfindungsgemäßen Peptide können in kompetitiven Immunoassays in markierter Form eingesetzt werden.

Vorzugsweise werden die Peptide gemäß der vorliegenden Erfindung zur Herstellung eines Antikörpers oder einer Antikörpermischung verwendet werden.

Des Weiteren werden die Peptide gemäß der vorliegenden Erfindung als Positivkontrolle bzw. als Standard für Konzentrationsbestimmungen in einem erfindungsgemäßen Verfahren angewendet.

Ein weiterer Aspekt der vorliegenden Erfindung betrifft einen Kit zur Bestimmung von felinem oder caninem proBNP, umfassend mindestens einen erfindungsgemäßen Antikörper oder mindestens eine erfindungsgemäße Antikörpermischung, Mittel zur qualitativen und/oder quantitativen Detektion einer Bindung des mindestens einen Antikörpers oder der mindestens einen Antikörpermischung an felinem oder caninem proBNP und gegebenenfalls erfindungsgemäße Peptide und/oder felines oder canines proBNP als Positiv-Kontrolle oder Standard für eine Konzentrationsbestimmung.

Erfindungsgemäß kann der Kit mindestens einen weiteren Antikörper umfassen.

Dieser zusätzliche Antikörper weist eine Avidität zum mindestens einen Antikörper oder aber auch zum mindestens einen Epitop auf.

Gemäß einer bevorzugten Ausführungsform ist der mindestens eine Antikörper und/oder der mindestens eine weitere Antikörper markiert.

Vorzugsweise umfasst die Markierung Enyzme, wie Peroxidasen, insbesondere Meerrettich-Peroxidase, Biotin, Fluoreszenzfarbstoffe, insbesondere Fluoreszein (FITC, DFTF), R-Phycoerythrin (PE), Peridiniumchlorophyll-Protein (PerCP) und Tandemkonjugate wie PE-Cy5 oder PE-Texas-Rot, Goldkolloid oder Radionuklide.

Ein weiterer Aspekt der vorliegenden Erfindung betrifft die Verwendung eines erfindungsgemäßen Kits in einem Verfahren zur Bestimmung von felinem oder caninem proBNP.

Mit dem Verfahren der vorliegenden Erfindung ist es möglich nicht nur proBNP und dessen Fragmente in Katzen und Hunden nachzuweisen sondern auch in anderen Säugetieren wie Pferden, Rindern, Elefanten, Mäusen (Swiss-Prot: P40753), Schweinen (Swiss-Prot: P07634), Ratten (Swiss-Prot: P13205), Kamelen (Swiss-Prot: Q6L7Z3) und Schafen (Swiss-Prot: 046541) und Fischen, wie Barschen (Swiss-Prot: Q805E8), Stören (Swiss-Prot: P83965) und Kugelfischen (Swiss-Prot: Q805D7).

Um proBNP in den oben genannten Tieren nachzuweisen, sind Antikörper, die an mindestens einem Epitop in einem Aminosäurebereich von Aminosäurerest 1 bis Aminosäurerest 80 des entsprechenden proBNPs binden, bevorzugt. Insbesondere sind Antikörper bevorzugt, die an mindestens einem Epitop umfassend die Aminosäurebereiche 1-15, 15-30, 20-30, 25-35, 30-40, 35-50, 35-55, 45-55, 50-70, 60-70, 60-80 und 70-80 binden. Die folgenden besonders bevorzugten spezifischen Epitope wurden ebenfalls mit dem der vorliegenden Erfindung zugrunde liegenden Schema aufgefunden (siehe Beispiele).

**Tabelle 1**

| | ***Swiss-Prot Nr.*** | ***AS-Bereich*** |
|---|---|---|
| Maus | P40753 | 1-15, 20-30, 50-70 |
| Schwein | P07634 | 20-30, 35-50, 60-70 |
| Ratte | P13205 | 1-15, 30-40, 45-55, 60-80 |
| Kamel | Q6L7Z3 | 20-30, 55-65, 70-80 |
| Schaf | 046541 | 1-15, 20-30, 45-55, 60-80 |
| Barsch | Q805E8 | 15-30, 35-50, 70-80 |
| Stör | P83965 | 1-20, 25-35, 70-80 |
| Kugelfisch | Q805D7 | 15-30, 35-55, 60-80 |

Die bevorzugten Längen der Epitope sind, wie oben für Hunde und Katzen angegeben, auch für die oben genannten Tiere gegeben.

Die vorliegende Erfindung ist weiters durch die folgenden Beispiele und Figuren veranschaulicht, ohne jedoch auf diese beschränkt zu sein.

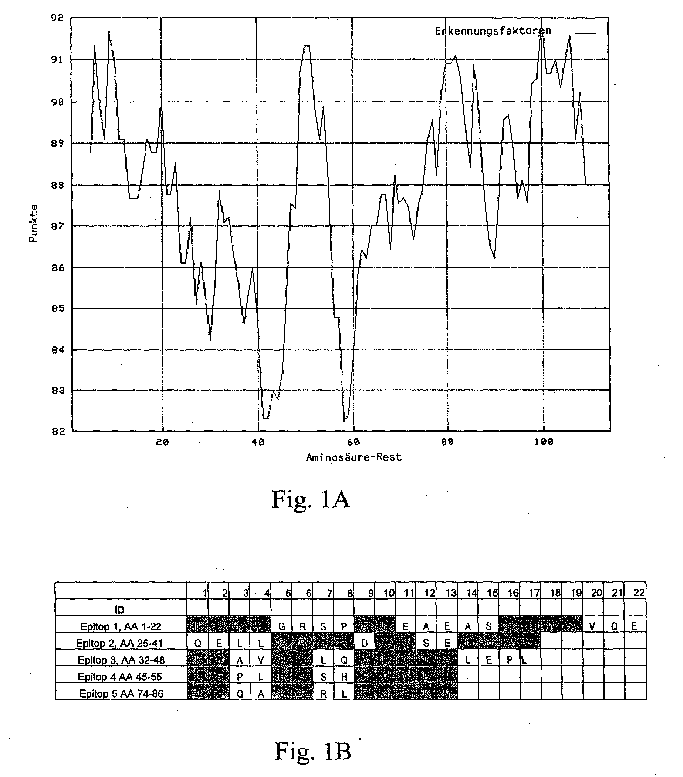

Fig.1 zeigt Epitop-Erkennungsfaktoren (Recognition factors) entsprechend dem Programm ProtScale der caninen proBNP-Aminosäuresequenz (Fig.1A) und Aminosäuresequenzen der erfindungsgemäßen caninen proBNP-Epitope (Fig.1B).

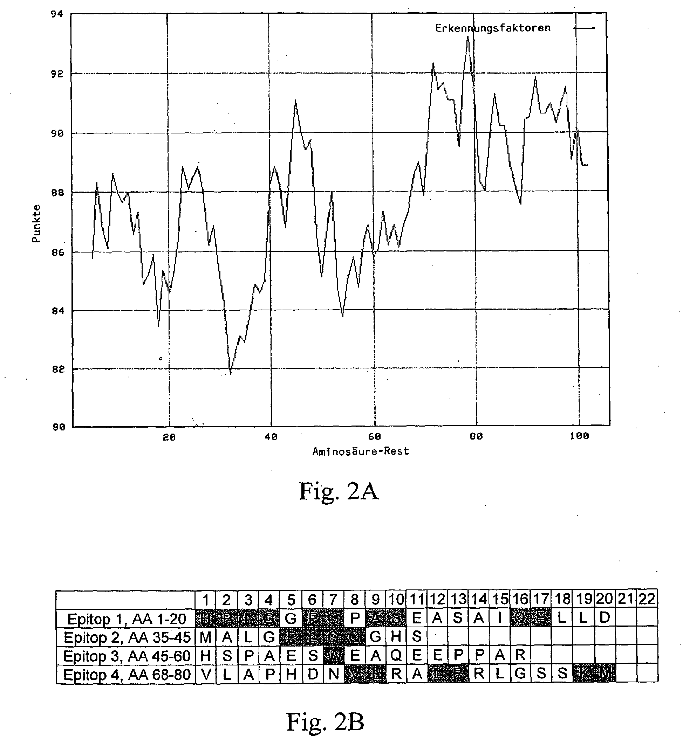

Fig.2 zeigt Epitop-Erkennungsfaktoren (Recognition factors) der felinen proBNP-Aminosäuresequenz berechnet mit ProtScale (Fig.2A) und Aminosäuresequenzen der erfindungsgemäßen felinen proBNP-Epitope (Fig.2B).

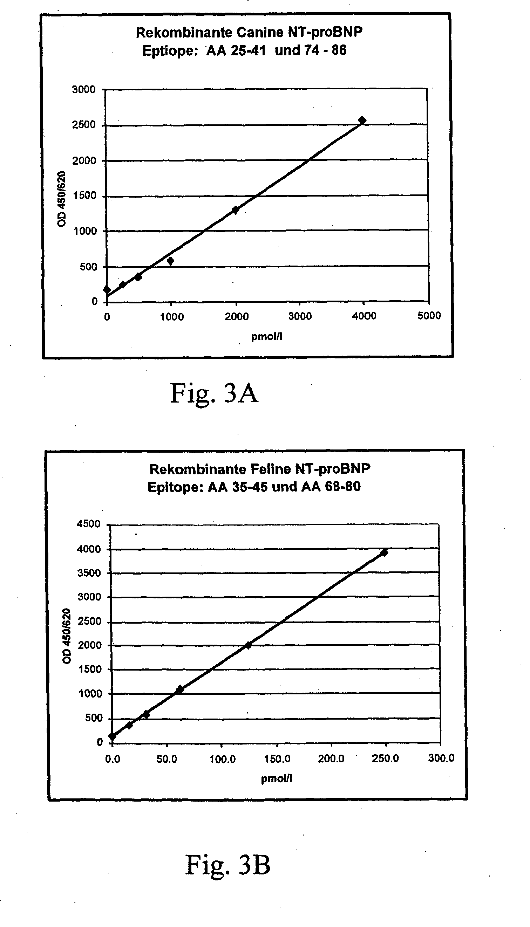

Fig.3 zeigt Standardkurven basierend auf ELISA-Untersuchungen unter Verwendung von erfindungsgemäßen Antikörpern mit caninem (Fig.3A) und felinem (Fig.3B) proBNP. Es konnte eindrucksvoll gezeigt werden, dass die proBNP-Bestimmung mit den erfindungsgemäßen Antikörpern über einen weiten Konzentrationsbereich linear verläuft.
Fig.4 zeigt die Bestimmung von proBNP in 47 kranken und 28 gesunden Katzen. Die Konzentration von proBNP in den Proben ermöglicht es die Schwere der Krankheit zu bestimmen. FAT - Feline atriale Thrombose, HKMP - hypertrophe Kardiomyopathie, LVH - linksventrikuläre Hyertrophie.

### BEISPIELE:

### Beispiel 1: Herstellung der Antikörper

Epitope können beispielsweise durch Berechnung mit ProtScale (http://www.expasy.org/tools/protscale.html) nach dem Algorithmus von Fraga S. ("Theoretical prediction of protein antigenic determinants from amino acid sequences." , 1982; Can. J. Chem. 60:2606-2610) bestimmt werden.

Die Peptidfragmente wurden aus denjenigen Bereichen der Aminosäuresequenz des felinen oder caninen Nt-proBNP ausgewählt bei denen ein Maximum der Epitop-Erkennungsfaktoren (entsprechend den Ergebnissen des Protscale-Programms) erhalten wurden, da diese Epitope sich als besonders immunogen herausgestellt haben und leicht zugänglich für Antikörper sind. Die ausgewählten Peptide des felinen oder caninen proBNP wurden chemisch synthetisiert und an ein geeignetes Träger-Protein (z.B. KLH) konjugiert.

Je ein an KLH konjugiertes Peptid/Epitop wurde drei Schafen injiziert. Jedes Schaf erhielt für die erste Immunisierung 0.5 mg des entsprechenden Antigens vermischt mit Freund'schem Adjuvans (Guildhay, UK) und BCG (Bacillus Calmette Guerlin) und 0.25 mg der Immunogene für das weitere Steigern der Immunantwort.

Erfindungsgemäß hat sich gezeigt, dass der Einsatz von polyklonalen Antikörpern sehr gute und reproduzierbare Ergebnisse liefert. Dennoch ist der Einsatz von monoklonalen Antikörpern in einem Verfahren wie in der vorliegenden Erfindung beschrieben ebenfalls möglich. Monoklonale Antikörper gegen Peptide/Epitope des felinen oder caninen proBNP können durch dem Fachmann bekannte Standardmethoden hergestellt werden (siehe hierzu beispielsweise Köhler G und Milstein C, Nature, 1975, 256:495-497).

### Beispiel 2: Bestimmung der Antikörperreaktivität mittels ELISA

Die Reaktivität von Antikörpern bzw. Seren gegen Peptide/Epitope des proBNP's, welche aus dem Blut der Schafe gewonnen werden, wurde mit Hilfe eines ELISA-Tests untersucht. Zunächst wurden die Mikrotiterplatten über Nacht bei 4°C mit Streptavidin (0.5 µg/ml, 200 µl pro Well) beschichtet, gewaschen, mit 1% BSA in 0,1 M PBS pH 7,5 enthaltend 0.25 % Tween 20 blockiert, nochmals gewaschen und für 3 h bei 4°C mit synthetischen proBNP Peptidsequenzen konjugiert an Biotin (0.25 µg/ml, 200 µl pro Well) inkubiert. Nach einem weiteren Waschschritt wurden die Serum-Proben mit 0,1 M Phosphatpuffer enthaltend 3% BSA 1:1000/1:10000/1:100000 verdünnt und auf die Mikrotiterplatte aufgebracht. Die Bindung der Antikörper an den Peptiden/Epitopen auf der Platte wurde durch die Zugabe von Anti-Schaf-IgG Antikörpern, welche mit Meerrettich-Peroxidase konjugiert sind, und von einer Substratlösung umfassend TMB (Tetramethylbenzidin) festgestellt. Die Reaktion der Meerrettich-Peroxidase mit TMB wurde durch die Zugabe von 0,9% Schwefelsäure gestoppt. Die Farbentwicklung wurde mit einem Photometer, welches in der Lage ist Mikrotiterplatten zu analysieren, überwacht.

### Beispiel 3: Nt-proBNP Messung in Proben gesunder und kranker Tiere

In die Wells einer Mikrotierplatte beschichtet mit einem der erfindungsgemäßen Antikörper wurden 20µl felines oder canines Serum pipettiert und mit 200µl eines zweiten weiteren, Peroxidase markierten erfindungsgemäßen Antikörper in 0.1M Phosphatpuffer pH 7 bei Raumtemperatur für 4-16h inkubiert. Anschließend wurde die Mikrotierplatte mit 5 x 300 µl 0.1M Phosphatpuffer pH 7 mit 0.1% Triton X100 gewaschen und 200µl Tetramethylbenzidin als Substrat zugesetzt. Nach 20-30 Minuten Farbentwicklung wird die Reaktion durch Zusatz von 50µl 0.9% Schwefelsäure gestoppt und die Farbintensität, welche direkt proportional zur Menge an Nt-proBNP ist, mit einem Mikrotierplatten-Photometer gemessen. Die genaue Konzentration wird durch Vergleich mit einer Eichkurve aus rekombinantem felinen oder caninen Nt-proBNP bestimmt.

Beispielhaft wurde die Konzentration von Nt-pro-BNP in 8 gesunden und in 15 herzkranken Hunden mit Hilfe von Antikörpern gegen Epitope im Bereich der Aminosäuren 25-41 und 74-86 des caninen Nt-proBNPs bestimmt (Tabelle 2):

**Tabelle 2**

| Gesundheitszustand | Nr. | canines Nt-proBNP |
|---|---|---|
| gesund | | *pmol*/*l* |
| | 1 | 862 |
| | 2 | 1060 |
| | 3 | 753 |
| | 4 | 531 |
| | 5 | 980 |
| | 6 | 674 |
| | 7 | 695 |
| | 8 | 1010 |
| | | |
| herzkrank | | |
| | 43 | 2460 |
| | 44 | 1950 |
| | 45 | 2140 |
| | 46 | 2170 |
| | 47 | 1480 |
| | 48 | 1560 |
| | 49 | 1390 |
| | 50 | 1450 |
| | 51 | 1520 |
| | 52 | 1790 |
| | 53 | 1310 |
| | 54 | 1140 |
| | 55 | 1975 |
| | 57 | 1720 |
| | 58 | 3020 |

Diese Ergebnisse zeigen, dass mit den Antikörpern der vorliegenden Erfindung die Konzentration von Nt-proBNP im Serum von Tieren effizient bestimmt werden kann und mit deren Ergebnisse eine Diagnose über den Gesundheitszustand erstellt werden kann bzw. der Verlauf einer Therapie überwacht werden kann.

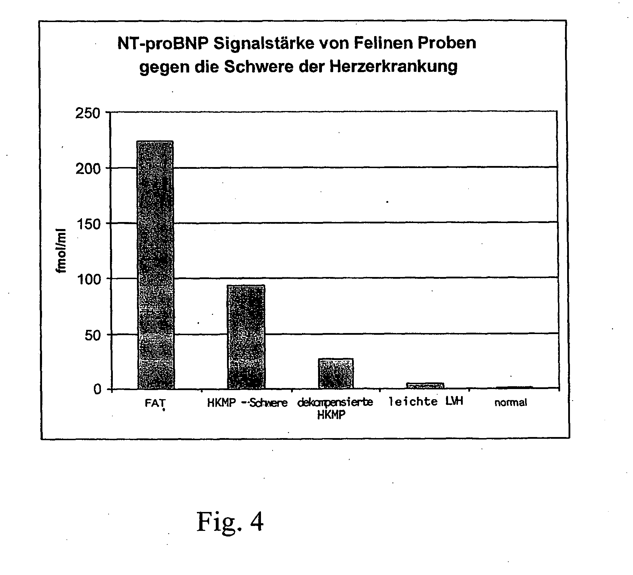

Fig.4 zeigt des Weiteren die Bestimmung von Nt-proBNP in 47 kranken und 28 gesunden Katzen. Dabei wurde festgestellt, dass die Konzentration von detektiertem Nt-proBNP in direkten Zusammenhang mit der Schwere der Herzerkrankung steht. Bei diesen Versuchen wurden Antikörper die Epitope im Bereich umfassend die Aminosäuren 35 bis 45 und 68 bis 80 binden eingesetzt. Die erhaltenen Ergebnisse bestätigen zahlreiche Veröffentlichungen, in denen ein ähnlicher Zusammenhang postuliert wurde.

### Beispiel 4: Kreuzreaktivität

Rekombinantes felines, canines und humanes Nt-proBNP wurden auf Mikrotierplatten beschichtet (250ng/ml, 200µl/well, über Nacht Raumtemperatur). Die Platten wurden anschließend gewaschen, und mit einer Verdünnung der anti-humanen, anti-felinen und anti-caninen Antisera (10-100µg/ml, in 0.1M Phosphatbuffer pH 7) in Kontakt gebracht. Die Menge gebundener Antikörper wurde nach einem Waschschritt mit einem geeigneten sekundären Antikörper (Peroxidase markierter Anti-Schaf Antikörper), gemessen. Dabei zeigte sich, dass die jeweiligen Antikörper sehr gut mit den entsprechenden Nt-proBNP Molekülen reagierten (also antifeliner Antiserum mit felinem Nt-proBNP) aber überraschenderweise nicht oder in sehr geringem Ausmass mit den Nt-proBNP Molekülen der jeweiligen anderen Spezies.

Es konnte gezeigt werden, dass die Antikörper, die gegen Epitope des felinen Nt-proBNP erzeugt wurden, eine hohe Spezifität aufweisen und nur in einem sehr geringen Ausmaß an die entsprechende humane Sequenz binden kann. Da bei der Messung der Antikörper-Spezifität Nt-proBNP als gesamtes Polypeptid als Bindungspartner eingesetzt wurde und nicht die Peptide, die für die Herstellung der Antikörper verwendet wurden, konnte eindrucksvoll gezeigt werden, dass die Antikörper gegen feline Epitope des Nt-proBNP keine Kreuzreaktion über den gesamten Sequenzbereich des humanen Nt-proBNP aufweisen. Eine Ausnahme bildet lediglich der Antikörper der im Bereich der Aminosäuren 1 bis 20 des felinen Nt-proBNP bindet. Dieser Antikörper zeigt bei der Bindung an felinem Nt-proBNP nur eine doppelt so hohe relative Reaktivität als bei der Bindung an humanen Nt-proBNP.

Des Weiteren konnte gezeigt werden, dass Antikörper gegen humane Epitope des Nt-proBNP ebenfalls eine geringe Reaktivität gegenüber felinem Nt-proBNP aufweisen. Somit konnte eindrucksvoll gezeigt werden, dass Antikörper, die gegen Epitope des humanen Nt-proBNP gerichtet sind nicht an felinem Nt-proBNP binden können und somit nicht zur Bestimmung von Nt-proBNP in Katzen herangezogen werden können (siehe Tabelle 3).

**Tabelle 3**

| Antiserum Nr. | Antikörper-Spezifität | Relative Reaktivität | Rel. Reaktivität gegenüber den entsprechenden humanen Sequenzen |
|---|---|---|---|
| S2189 | AA 1-20 felin | 2.3 | 1.2 |
| S2190 | AA 45-55 felin | 3.7 | 0.01 |
| S2191 | AA 25-35 felin | 1.0 | 0.2 |
| S2192 | AA 60-80 felin | 4.2 | 0.3 |
| S2072 | AA 8-29 human | 0.4 | ------ |
| S2104 | AA 32-57 human | 0.25 | ------ |
| S2102 | AA 60-80 human | 1.7 | ------ |

Die Kreuzreaktivität wurde ebenfalls mit Antikörpern gegen Epitope des caninen Nt-proBNP und mit Antikörpern gegen Epitope des humanen Nt-proBNP getestet. Auch bei caninen Sequenzen konnte ein vergleichbares Ergebnis wie bei den Untersuchungen mit felinen Sequenzen erzielt werden (siehe Tabelle 4).

**Tabelle 4**

| Antiserum Nr. | Antikörper-Spezifität | Relative Reaktivität | Rel. Reaktivität gegenüber den entsprechenden humanen Sequenzen |
|---|---|---|---|
| S2195 | AA 1-22 canin | 2.2 | 1.1 |
| S2196 | AA 25-41 canin | 6.3 | 0.2 |
| S2197 | AA 55-65 canin | 1.0 | 0.03 |
| S2198 | AA 74-86 canin | 1.9 | 0.6 |
| S2072 | AA 8-29 human | 0.1 | ------ |
| S2104 | AA 32-57 human | 0.3 | ------ |
| S2102 | AA 60-80 human | 1.5 | ----- |

## Patentansprüche

1. Verfahren zur Bestimmung von felinem oder caninem proBNP umfassend die Schritte:
- Bereitstellen einer felinen oder caninen Probe,
- Inkontaktbringen der Probe mit mindestens einem Antikörper, der bei der Bestimmung von felinem proBNP an mindestens ein Epitop im Bereich der Aminosäuren 20 bis 42 oder im Bereich der Aminosäuren 57 bis 80 des felinen proBNP und bei der Bestimmung von caninem proBNP an mindestens ein Epitop im Bereich der Aminosäuren 25 bis 41 oder im Bereich der Aminosäuren 55 bis 65 oder im Bereich der Aminosäuren 74 bis 86 des caninen proBNP bindet, und
- Bestimmen der Anwesenheit und/oder Konzentration des in der Probe vorhandenen felinen oder caninen proBNP.

2. Verfahren nach Anspruch 1, **dadurch gekennzeichnet, dass** bei der Bestimmung von felinem proBNP der mindestens eine Antikörper an mindestens ein Epitop im Bereich der Aminosäuren 25 bis 35 oder im Bereich der Aminosäuren 60 bis 80 des felinen proBNP bindet.

3. Verfahren nach Anspruch 1 oder 2, **dadurch gekennzeichnet, dass** an den mindestens einen Antikörper oder an das mindestens eine Epitop mindestens ein weiterer Antikörper bindet.

4. Verfahren nach einem der Ansprüche 1 bis 3, **dadurch gekennzeichnet, dass** der mindestens eine Antikörper und/oder der mindestens eine weitere Antikörper markiert ist.

5. Verfahren nach Anspruch 4, **dadurch gekennzeichnet, dass** der mindestens eine Antikörper und/oder der mindestens eine weitere Antikörper mit Peroxidase, insbesondere Meerrettich-Peroxidase, Biotin, Fluoreszenzfarbstoff, Goldkolloid oder Radionuklide markiert ist.

6. Verfahren nach einem der Ansprüche 1 bis 5, **dadurch gekennzeichnet, dass** der mindestens eine Antikörper oder der mindestens eine weitere Antikörper an eine Festphase gebunden ist.

7. Verfahren nach einem der Ansprüche 1 bis 6, **dadurch gekennzeichnet, dass** die Bestimmung von felinem oder caninem proBNP durch ein Verfahren ausgewählt aus der Gruppe bestehend aus Radioimmunassay, Immunbindungsassay, Westernblot, Immunhistochemie, Enzymimmunoassay, Lateral Flow Device (LFD, Teststreifen) und Kombinationen davon, erfolgt.

8. Antikörper oder Antikörpermischung, **dadurch gekennzeichnet, dass** dieser/diese an mindestens ein Epitop im Bereich der Aminosäuren 20 bis 42 oder im Bereich der Aminosäuren 57 bis 80 des felinen proBNP, vorzugsweise im Bereich der Aminosäuren 25 bis 35 oder im Bereich der Aminosäuren 60 bis 80 des felinen proBNP, bindet.

9. Antikörper oder Antikörpermischung, **dadurch gekennzeichnet, dass** dieser/diese an mindestens ein Epitop im Bereich der Aminosäuren 25 bis 41 oder im Bereich der Aminosäuren 55 bis 65 oder im Bereich der Aminosäuren 74 bis 86 des caninen proBNP bindet.

10. Peptid, bestehend aus den Aminosäuren 20 bis 42 und/oder den Aminosäuren 57 bis 80, vorzugsweise den Aminosäuren 25 bis 35 und/oder den Aminosäuren 60 bis 80, des felinen proBNP.

11. Peptid, bestehend aus den Aminosäuren 25 bis 41 und/oder den Aminosäuren 55 bis 65 und/oder den Aminosäuren 74 bis 86 des caninen proBNP.

12. Verwendung eines Peptids nach Anspruch 10 oder 11 als Positiv-Kontrolle oder als Standard für eine Konzentrationsbestimmung in einem Verfahren nach einem der Ansprüche 1 bis 7.

13. Kit zur Bestimmung von felinem oder caninem proBNP umfassend mindestens einen Antikörper oder mindestens eine Antikörpermischung nach Anspruch 8 oder 9, Mittel zur qualitativen und/oder quantitativen Detektion einer Bindung des mindestens einen Antikörpers oder der mindestens einen Antikörpermischung an felinem oder caninem proBNP und gegebenenfalls Peptide nach Anspruch 10 oder 11 und/oder felines oder canines proBNP als Positiv-Kontrolle oder Standard für eine Konzentrationsbestimmung.

14. Kit nach Anspruch 13, **dadurch gekennzeichnet, dass** die Mittel zur qualitativen und/oder quantitativen Detektion einer Bindung des mindestens einen Antikörpers oder der mindestens einen Antikörpermischung an felinem oder caninem proBNP mindestens einen weiteren Antikörper umfassen.

15. Kit nach Anspruch 13 oder 14, **dadurch gekennzeichnet, dass** der mindestens eine Antikörper und/oder der mindestens eine weitere Antikörper markiert ist.

16. Kit nach einem der Ansprüche 13 bis 15, **dadurch gekennzeichnet, dass** der mindestens eine Antikörper und/oder der mindestens eine weitere Antikörper mit Peroxidase, insbesondere Meerrettich-Peroxidase, Biotin, Fluoreszenzfarbstoff, Goldkolloid oder Radionuklide markiert ist.

## Claims

1. A method for assaying feline or canine proBNP, comprising the following steps:
- providing a feline or canine sample;
- bringing the sample into contact with at least one antibody which, when assaying feline proBNP, binds to at least one epitope in the region comprising amino acids 20 to 42 or in the region comprising amino acids 57 to 80 of feline proBNP, and when assaying canine proBNP, binds to at least one epitope in the region comprising amino acids 25 to 41 or in the region comprising amino acids 55 to 65 or in the region comprising amino acids 74 to 86 of canine proBNP; and
- assaying the presence and/or concentration of feline or canine proBNP present in the sample.

2. The method according to claim 1, **characterized in that** when assaying feline proBNP, the at least one antibody binds to at least one epitope in the region comprising amino acids 25 to 35 or in the region comprising amino acids 60 to 80 of feline proBNP.

3. The method according to claim 1 or 2, **characterized in that** at least one further antibody binds to the at least one antibody or to the at least one epitope.

4. The method according to one of claims 1 to 3, **characterized in that** the at least one antibody and/or the at least one further antibody is labelled.

5. The method according to claim 4, **characterized in that** the at least one antibody and/or the at least one further antibody is labelled with peroxidase, in particular horseradish peroxidase, biotin, fluorescent dye, colloidal gold or radionuclides.

6. The method according to one of claims 1 to 5, **characterized in that** the at least one antibody or the at least one further antibody is bound to a solid phase.

7. The method according to one of claims 1 to 6, **characterized in that** the feline or canine proBNP is assayed using a method selected from the group consisting of radioimmunoassay, immune binding assay, Western blot, immunohistochemistry, enzyme immunoassay, a lateral flow device (LFD, test strips), and combinations thereof.

8. An antibody or antibody mixture, **characterized in that** it binds to at least one epitope in the region comprising amino acids 20 to 42 or in the region comprising amino acids 57 to 80 of feline proBNP, preferably in the region of amino acids 25 to 35 or in the region of amino acids 60 to 80 of feline proBNP.

9. An antibody or an antibody mixture, **characterized in that** it binds to at least one epitope in the region comprising amino acids 25 to 41 or in the region comprising amino acids 55 to 65 or in the region comprising amino acids 74 to 86 of canine proBNP.

10. A peptide consisting of amino acids 20 to 42 and/or amino acids 57 to 80, preferably amino acids 25 to 35 and/or amino acids 60 to 80, of feline proBNP.

11. A peptide consisting of amino acids 25 to 41 and/or amino acids 55 to 65 and/or amino acids 74 to 86 of canine proBNP.

12. Use of a peptide according to claim 10 or 11 as a positive control or as a standard for an assay of concentration in a method according to one of claims 1 to 7.

13. A kit for assaying feline or canine proBNP, comprising at least one antibody or at least one antibody mixture according to claim 8 or 9, means for the qualitative and/or quantitative detection of binding of the at least one antibody or the at least one antibody mixture to feline or canine proBNP, and optionally, peptides according to claim 10 or 11 and/or feline or canine proBNP as a positive control or as a standard for an assay of concentration.

14. The kit according to claim 13, **characterized in that** the means for qualitative and/or quantitative detection of binding of the at least one antibody or the at least one antibody mixture to feline or canine proBNP comprise at least one further antibody.

15. The kit according to claim 13 or 14, **characterized in that** the at least one antibody and/or the at least one further antibody is/are labelled.

16. The kit according to one of claims 13 to 15, **characterized in that** the at least one antibody and/or the at least one further antibody is/are labelled with peroxidase, in particular with horseradish peroxidase, biotin, fluorescent dye, colloidal gold or radionuclides.

## Revendications

1. Procédé pour la détermination du proBNP félin ou canin comprenant les étapes consistant à :
- préparer un échantillon félin ou canin,
- mettre en contact l'échantillon avec au moins un anticorps qui, lors de la détermination du proBNP félin, se lie à au moins un épitope dans le domaine des acides aminés 20 à 42 ou dans le domaine des acides aminés 57 à 80 du proBNP félin et, lors de la détermination du proBNP canin, se lie à au moins un épitope dans le domaine des acides aminés 25 à 41 ou dans le domaine des acides aminés 55 à 65 ou dans le domaine des acides aminés 74 à 86 du proBNP canin, et
- déterminer la présence et/ou la concentration du proBNP félin ou canin existant dans l'échantillon.

2. Procédé selon la revendication 1, **caractérisé en ce que**, lors de la détermination du proBNP félin, le au moins un anticorps se lie à au moins un épitope dans le domaine des acides aminés 25 à 35 ou dans le domaine des acides aminés 60 à 80 du proBNP félin.

3. Procédé selon la revendication 1 ou 2, **caractérisé en ce qu'**au moins un autre anticorps se lie au au moins un anticorps ou au au moins un épitope.

4. Procédé selon l'une des revendications 1 à 3, **caractérisé en ce que** le au moins un anticorps et/ou le au moins un autre anticorps est marqué.

5. Procédé selon la revendication 4, **caractérisé en ce que** le au moins un anticorps et/ou le au moins un autre anticorps est marqué avec de la peroxydase, en particulier de la peroxydase de raifort, avec de la biotine, avec un colorant fluorescent, avec de l'or colloïdal ou avec des radionucléides.

6. Procédé selon l'une des revendications 1 à 5, **caractérisé en ce que** le au moins un anticorps ou le au moins un autre anticorps est lié à une phase solide.

7. Procédé selon l'une des revendications 1 à 6, **caractérisé en ce que** la détermination du proBNP félin ou canin est réalisée par un procédé choisi dans le groupe constitué du radio-immunodosage, du dosage par liaison immunitaire, du buvardage de Western, de l'immunohistochimie, du dosage immunoenzymatique, d'un dispositif à flux latéral (LFD, lateral flow device, avec bandelettes) et des combinaisons de ceux-ci.

8. Anticorps ou mélange d'anticorps, **caractérisé en ce que** celui-ci/ceux-ci se lie(nt) à au moins un épitope dans le domaine des acides aminés 20 à 42 ou dans le domaine des acides aminés 57 à 80 du proBNP félin, de préférence dans le domaine des acides aminés 25 à 35 ou dans le domaine des acides aminés 60 à 80 du proBNP félin.

9. Anticorps ou mélange d'anticorps, **caractérisé en ce que** celui-ci/ceux-ci se lie(nt) à au moins un épitope dans le domaine des acides aminés 25 à 41 ou dans le domaine des acides aminés 55 à 65 ou dans le domaine des acides aminés 74 à 86 du proBNP canin.

10. Peptide constitué des acides aminés 20 à 42 et/ou des acides aminés 57 à 80, de préférence des acides aminés 25 à 35 et/ou des acides aminés 60 à 80, du proBNP félin.

11. Peptide constitué des acides aminés 25 à 41 et/ou des acides aminés 55 à 65 et/ou des acides aminés 74 à 86 du proBNP canin.

12. Utilisation d'un peptide selon la revendication 10 ou 11 comme contrôle positif ou comme standard pour une détermination de concentration dans un procédé selon l'une des revendications 1 à 7.

13. Kit pour la détermination du proBNP félin ou canin comprenant au moins un anticorps ou au moins un mélange d'anticorps selon la revendication 8 ou 9, des moyens pour la détection qualitative et/ou quantitative d'une liaison du au moins un anticorps ou du au moins un mélange d'anticorps au proBNP félin ou canin et le cas échéant de peptides selon la revendication 10 ou 11 et/ou le proBNP félin ou canin comme contrôle positif ou standard pour une détermination de concentration.

14. Kit selon la revendication 13, **caractérisé en ce que** les moyens pour la détection qualitative et/ou quantitative d'une liaison du au moins un anticorps ou du au moins un mélange d'anticorps au proBNP félin ou canin comprennent au moins un autre anticorps.

15. Kit selon la revendication 13 ou 14, **caractérisé en ce que** le au moins un anticorps et/ou le au moins un autre anticorps est marqué.

16. Kit selon l'une des revendications 13 à 15, **caractérisé en ce que** le au moins un anticorps et/ou le au moins un autre anticorps est marqué avec de la peroxydase, en particulier de la peroxydase de raifort, avec de la biotine, avec un colorant fluorescent, avec de l'or colloïdal ou avec des radionucléides.
